(19) Europäisches Patentamt
European Patent Office
Office européen des brevets

(11) **EP 3 639 813 B1**

(12) **EUROPEAN PATENT SPECIFICATION**

(45) Date of publication and mention
of the grant of the patent:
**12.01.2022 Bulletin 2022/02**

(51) Int Cl.:
*A61K 9/12* (2006.01)          *A61K 47/10* (2017.01)
*A24F 47/00* (2020.01)        *A61K 31/465* (2006.01)
*A61K 9/72* (2006.01)          *A24B 15/16* (2020.01)

(21) Application number: **19199645.3**

(22) Date of filing: **20.05.2015**

(54) **A LIQUID FORMULATION COMPRISING NICOTINE FOR AEROSOL ADMINISTRATION**

FLÜSSIGE FORMULIERUNG MIT NIKOTIN ZUR AEROSOLVERABREICHUNG

FORMULATION LIQUIDE CONTENANT DE LA NICOTINE POUR L'ADMINISTRATION D'AÉROSOLS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **21.05.2014 SE 1450609**

(43) Date of publication of application:
**22.04.2020 Bulletin 2020/17**

(62) Document number(s) of the earlier application(s) in
accordance with Art. 76 EPC:
**15722728.1 / 3 145 492**

(73) Proprietor: **McNeil AB
251 09 Helsingborg (SE)**

(72) Inventors:
 • **MUHAMMED, Salih Mushin
   S-265 74 Hyllinge (SE)**
 • **LINDELL, Katarina
   S-241 93 Eslöv (SE)**
 • **KELEPOURIS, Lee
   S-253 74 Helsingborg (SE)**

(74) Representative: **Carpmaels & Ransford LLP
One Southampton Row
London WC1B 5HA (GB)**

(56) References cited:
**WO-A1-2013/088230      GB-A- 2 030 862**

• **Anonymous: "Deutsche Liquid Basen 36 mg/ml",
Wayback.archive , 1 March 2014 (2014-03-01),
pages 1-2, XP002742437, Retrieved from the
Internet:
URL:http://wayback.archive.org/web/2014030
1184947/http://www.avoria-liquids.de/deuts
che-liquid-basen-36mg/ml [retrieved on
2015-07-15]**
• **ZHANG YAPING ET AL: "In vitro particle size
distributions in electronic and conventional
cigarette aerosols suggest comparable
deposition patterns", NICOTINE & TOBACCO
RESEARCH : OFFICIAL JOURNAL OF THE
SOCIETY FOR RESEARCH ON NICOTINE AND
TOBACCO,, vol. 15, no. 2, 1 February 2013
(2013-02-01), pages 501-508, XP009171613, ISSN:
1469-994X, DOI: 10.1093/NTR/NTS165**

EP 3 639 813 B1

**Description**

**Background of the Invention**

Over the years, various types of products have been developed, as an alternative means to smoking, to administer nicotine to a user to aid in smoking cessation (e.g., gums, lozenges, patches, nasal sprays, mouth sprays, and inhalators). For certain drugs, inhalation is considered as an efficacious route of administration due to the rapid absorption of the drug and avoidance of the first pass effect. For such drugs, a high plasma level can be achieved rapidly after inhalation of aerosol formulations. In the case of nicotine replacement therapy, it is believed that a rapid and sufficiently high nicotine plasma levels in the body is of high importance as tobacco smoking also delivers nicotine to the body in the form of inhalable aerosols.

Recently, e-cigarettes, electronic inhalators designed to look like a cigarette that generates aerosols usually containing nicotine, have become popular. These devices mimic the look and feel of a cigarette, but claim to avoid the dangers associated with inhaling burned tobacco smoke. In order to mimic cigarette smoke, these electronic inhalers often utilize liquid formulations (often containing glycerol) that, upon use, create aerosols that, like tobacco smoke, are visible upon exhalation by the user.

Although, many e-cigarettes formulations contain nicotine, their use as a means to quit smoking can be inadequate for various reasons, such as: (i) the nicotine concentration in the formulation can be too low, (ii) a considerable amount of nicotine can be lost by not being captured by the body, and (iii) given the visible aerosol they often generate, their use is not discreet and mimics the habit that the user desires to quit. Thus, there is a need to develop liquid formulation containing a high concentration of nicotine whose generated aerosol is less visible and is better retained in the oral and respiratory tracts.

GB2030862A relates to a composition for treating drug addiction, such as the smoking of nicotine-containing products or the taking of heroin, comprising an aerosol containing the active agent of the addiction, i.e. nicotine for tobacco smokers or morphine for heroin addicts.

"Deutsche Liquid Basen 36 mg/ml" (anonymous) relates to liquid bases for e-cigarettes.

Zhang Yaping et al., "In vitro particle size distributions in electronic and conventional cigarette aerosols suggest comparable deposition patterns" NICOTINE & TOBACCO RESEARCH: OFFICIAL JOURNAL OF THE SOCIETY FOR RESEARCH ON NICOTINE AND TOBACCO, vol. 15, no. 2, 1 February 2013, pages 501-508, relates to aerosol particle sizes and their effect on deposition patterns in vapers (users of electronic cigarettes) and bystanders.

**Summary of the Invention**

The present invention relates to a liquid formulation comprising:

(i) 29.4 percent by weight of water;
(ii) 68.6 percent by weight of propylene glycol; and
(iii) 2 percent by weight of nicotine.

The present invention relates to a liquid formulation comprising:

(i) 39.2 percent by weight of water;
(ii) 58.8 percent by weight of propylene glycol; and
(iii) 2 percent by weight of nicotine.

The present invention relates to a liquid formulation comprising:

(i) 28.8 percent by weight of water;
(ii) 67.2 percent by weight of propylene glycol; and
(iii) 4 percent by weight of nicotine.

The present invention relates to a liquid formulation comprising:

(i) 38.4 percent by weight of water;
(ii) 57.6 percent by weight of propylene glycol; and
(iii) 4 percent by weight of nicotine.

The present invention relates to a liquid formulation comprising:

(i) 29.10 percent by weight of water;
(ii) 67.90 percent by weight of propylene glycol; and
(iii) 3.00 percent by weight of nicotine.

The present invention relates to a liquid formulation comprising:

(i) 28.65 percent by weight of water;
(ii) 66.85 percent by weight of propylene glycol; and
(iii) 4.50 percent by weight of nicotine.

The present invention relates to a liquid formulation comprising:

(i) 38.80 percent by weight of water;
(ii) 58.20 percent by weight of propylene glycol; and
(iii) 3.00 percent by weight of nicotine.

The present invention relates to a liquid formulation comprising:

(i) 38.20 percent by weight of water;
(ii) 57.30 percent by weight of propylene glycol; and
(iii) 4.50 percent by weight of nicotine.

The present invention relates to a liquid formulation comprising:

(i) 28.20 percent by weight of water;
(ii) 65.80 percent by weight of propylene glycol; and
(iii) 6 percent by weight of nicotine.

In one aspect which is not claimed, the present disclosure features a method of administering nicotine or a salt thereof to a human, wherein the method includes inhaling an aerosol of a liquid formulation, the liquid formulation comprising: (i) at least 12 percent by weight of water; (ii) at least 70 percent by weight of propylene glycol; and (iii) at least 2 percent by weight of said nicotine or a salt thereof; wherein the liquid formulation includes no more than 5 percent by weight of glycerol and no more than 5 percent by weight of ethanol.

In another aspect which is not claimed, the present disclosure features an aerosol-generating device comprising a reservoir containing a liquid formulation, wherein the liquid formulation comprising: (i) at least 12 percent by weight of water; (ii) at least 70 percent by weight of propylene glycol; and (iii) at least 2 percent by weight of said nicotine or a salt thereof; wherein the liquid formulation includes no more than 5 percent by weight of glycerol and no more than 5 percent by weight of ethanol and wherein the aerosol-generating device is arranged and configured to generate an aerosol of said liquid formulation.

In still another aspect which is not claimed, the present disclosure features a reservoir containing a liquid formulation, wherein the liquid formulation comprising: (i) at least 12 percent by weight of water; (ii) at least 70 percent by weight of propylene glycol; and (iii) at least 2 percent by weight of said nicotine or a salt thereof; wherein the liquid formulation includes no more than 5 percent by weight of glycerol and no more than 5 percent by weight of ethanol and wherein the reservoir is arranged and configured to attach to an aerosol-generating device to provide fluid communication for such liquid formulation from the reservoir to the aerosol- generating device.

Other features and advantages of the present invention will be apparent from the detailed description of the invention and from the claims.

## Detailed Description of the Invention

It is believed that one skilled in the art can, based upon the description herein, utilizes the present invention to its fullest extent. The following specific embodiments can be construed as merely illustrative, and not limitative of the remainder of the disclosure in any way whatsoever.

Unless defined otherwise, all technical and scientific terms used herein have the same meaning as commonly understood by one of ordinary skill in the art to which the invention belongs. As used herein, all percentages are by weight unless otherwise specified.

Liquid Formulation

The liquid formulation includes no glycerol. As discussed above, the presence of glycerol in the formulation will result in a more visible aerosol form of the liquid formulation. Further, glycerol has a higher boiling point than propylene glycol, which would thus potentially raise the boiling point of the liquid formulation. Still further, glycerol can crystalize at low temperatures.

The liquid formulation includes no ethanol. The inclusion of ethanol is not desired because of both the risk of separation due to rapid evaporation when heating the formulation to create an aerosol as well as the desire of certain users to avoid the intake of ethanol into their body.

As discussed below in the examples, Applicants have found that the claimed concentration of water lowers the boiling point of the liquid formulation while still maintaining at an acceptably low water activity to inhibit microbial activity (e.g., a water activity of less than 0.6). In one embodiment, the boiling point of the liquid formulation is less than 125°C, such as from 105°C to 125°C (e.g., when measured using the method recited in the European Pharmacopoeia 8th Edition 2.2.12).

Maintaining the boiling point above 105°C (e.g., by maintaining at least 70 percent by weight of propylene glycol) but below 125°C not only allows for the creation of the aerosol at a temperature less than the temperature needed to create an aerosol with just propylene glycol and/or glycerol, but it also may help prevent the fractionation of water and propylene glycol during such process which may help maintain a uniform nicotine concentration within the resulting aerosol.

In one embodiment, the viscosity of the liquid formulation is less than 75mPa.s, such as less than 50mPa.s, such as less than 30mPa.s (e.g., when measured using the method recited in the European Pharmacopoeia 8th Edition 2.2.8 at 20°C).

The liquid formulation may be manufactured by simple mixing of all the ingredients. Liquid ingredients are generally miscible with each other, while any solid ingredients may need to be dissolved in the mixture. Gentle heating of the constituent can help to dissolve solid components more rapidly.

Nicotine

The liquid formulation contains at least 2 percent by weight of nicotine. In one embodiment, the liquid formulation includes nicotine (i.e., the free base of nicotine). Examples of nicotine salts include, but are not limited to, formic (2:1), acetic (3:1), propionic (3:1), butyric (3:1), 2-methylbutyric (3:1), 3-methylbutynic (3:1), valeric (3:1), lauric (3:1), palmitic (3:1), tartaric (1:1) and (2:1), citric (2:1), malic (2:1), oxalic (2:1), benzoic (1:1), gentisic (1:1), gallic (1:1), phenylacetic (3:1), salicylic (1:1), phthalic (1:1), picric (2:1), sulfosalicylic (1:1), tannic (1:5), pectic (1:3), alginic (1:2), hydrochloric (2:1), chloroplatinic (1:1), silcotungstic (1:1), pyruvic (2:1), glutamic (1:1), and aspartic (1:1) salts of nicotine. The use of such salts may lower the pH to potentially reduce irritation for liquid formulations containing a high concentration of nicotine (not according to the invention).

As discussed above, the liquid formulation contains at least 2 percent by weight of nicotine, such as from 2 to 6 percent, such as 3 to 6 percent by weight of nicotine or a salt thereof. Benefits of such a high concentration of nicotine include reducing the amount of vapor needed to deliver a specified amount of nicotine and reducing the number of inhalations needed to release the dose.

Aerosol-generating Device

Numerous commercially available aerosol-generating devices have been developed to administer a liquid formulation as an aerosol to a user. In one embodiment, the aerosol-generating device is disposable after the liquid formulation stored within a reservoir within the device is exhausted. See, e.g., Njoy® King electronic cigarettes (Njoy, Scottsdale, Arizona) and Vype™ (CN Creative, Manchester, England). In one embodiment, the aerosol-generating device comprises a reservoir for the liquid formulation that is replaceable once the liquid formulation is exhausted. See, e.g., Vuse™ digital vapor cigarettes (RJ Reynolds Vapor Company, Winston-Salem, NC) and Vype™ Reload (CN Creative, Manchester, England) and US Patent No. 2013/0192617. In one embodiment, the aerosol-generating device thermally creates the aerosol. See, e.g., US Patent Nos. 2014/0000638 and 2013/0192617 and European Patent No. 1618803. In one embodiment, the aerosol-generating device creates the aerosol through mechanical formation, such as a nebulizer (e.g., ultrasonic and pneumatic nebulizers such has disclosed in US Patent No. 8,127,772).

Optical Density of Aerosol

The optical density of the aerosol may be determined using the method of Example 5 of this application ("Optical Density"). In one embodiment, the Optical Density of the aerosol is less than 0.05, such as less than 0.025, such as less than 0.01, such as less than 0.005, such as less than 0.001 where such Optical Density is measured at 5, 10, 15, and/or 20 seconds. In one embodiment, the Optical Density of the aerosol is less than 0.01 at 10 seconds, such as less than 0.005 at 5 seconds, such as less than 0.005 at 10 seconds, such as less than 0.005 at 5 seconds.

Use of Aerosol of Liquid Formulation

The method, reservoir, and/or device (not according to the invention) is used as an aid to smoking cessation, including but not limited to reliving and/or preventing withdrawal symptoms and/or reducing smoking cravings (e.g., for a user that is trying to stop smoking or reduce the number of cigarettes smoked). The method includes the administration of from about 0.01mg to about 0.25 mg of nicotine per inhalation (e.g., the device utilizing the liquid formulation is arranged and configured to generate an aerosol of the liquid formulation containing from about 0.05 mg to about 0.15 mg of nicotine per inhalation.

Examples

Specific embodiments of the present invention are illustrated by way of the following examples. This invention is not confined to the specific limitations set forth in these examples. Examples 1A to 1E, 1G to 1I, 2A to 2D, 2G, 2H, 2K, 2L, 3A to 3H, 3N, and 3O are provided for reference.

Example 1: Measurement of Boiling Point

Various liquid formulations including nicotine, water, and/or propylene glycol are setforth below in Table 1. These examples were manufactured as follows. First, all the ingredients were weighed. The Nicotine free base was added to the water, and then propylene glycol was added to nicotine/water solution followed by simple mixing to obtain homogenous liquid formulation. The boiling points for each of the formulations were measured and are recorded in Table 1.

Table 1

|  | Propylene Glycol (w/w %) | Water (w/w %) | Nicotine (w/w %) | Boiling Point (°C)* |
|---|---|---|---|---|
| Example 1A | 100 | 0 | 0 | 188 |
| Example 1B | 97 | 0 | 3 | 147.2 |
| Example 1C | 89.1 | 9.9 | 1 | 122.4 |
| Example 1D | 90 | 10 | 0 | 121.7 |
| Example 1E | 77.6 | 19.6 | 2 | 116.0 |
| Example 1F | 58.8 | 39.2 | 2 | 108.5 |
| Example 1G | 39.2 | 58.8 | 2 | 105.6 |
| Example 1H | 19.6 | 77.6 | 2 | 102.0 |
| Example 1I | 0 | 98 | 2 | 99.2 |
| * Boiling point was measured using the method described in the European Pharmacopoeia 8th Edition 2.2.8 | | | | |

Thus, as indicated in Table 1, even the mere addition of 10% water reduced the boiling point of the liquid formulation from 188 to 121.7 °C. Such a reduction allows the liquid formulation to form an aerosol at a much lower temperature.

Example 2: Water Activity

Water activity, or $a_w$, is the partial vapor pressure of water in a substance divided by the standard state partial vapor pressure of water. Water activity is based on a scale of 0 to 1.0 with pure water having a water activity of 1.0. Usually products that contain lower percent moisture have lower water activity. Water activity is an important characteristic to determine the antimicrobial properties of the formulation. Since yeast, molds, and bacteria require a certain amount of available water to support growth, designing a formulation with a water activity below 0.6 provides an effective control against such growth.

Various liquid formulations including nicotine, water, and/or propylene glycol are setforth below in Table 2.

These examples were manufactured as set forth in Example 1. The water activity ($a_w$) of these formulations was measured using an AquaLab 4TEV (Pullman, WA USA) chilled mirror water activity instrument and recorded in Table 2.

Table 2

|  | Propylene Glycol (w/w %) | Water (w/w %) | Nicotine (w/w %) | Water Activity* |
|---|---|---|---|---|
| Example 2A | 100 | 0 | 0 | 0.18 |
| Example 2B | 89.1 | 9.9 | 1 | 0.44 |
| Example 2C | 87.3 | 9.7 | 3 | 0.43 |
| Example 2D | 78.4 | 19.6 | 2 | 0.57 |
| Example 2E | 68.6 | 29.4 | 2 | 0.69 |
| Example 2F | 58.8 | 39.2 | 2 | 0.77 |
| Example 2G | 88.2 | 9.8 | 2 | 0.35 |
| Example 2H | 78.4 | 19.6 | 2 | 0.54 |
| Example 21 | 68.6 | 29.4 | 2 | 0.66 |
| Example 2J | 58.8 | 39.2 | 2 | 0.74 |
| Example 2K | 86.4 | 9.6 | 4 | 0.35 |
| Example 2L | 76.8 | 19.2 | 4 | 0.54 |
| Example 2M | 67.2 | 28.8 | 4 | 0.67 |
| Example 2N | 57.6 | 38.4 | 4 | 0.75 |

* The water activity was measured by an AquaLab 4TEV apparatus. Formulations containing less than 20% w/w of water were able to maintain a water activity of less than 0.6. Further, as seen when comparing below Examples 2B and 2C, changing the concentration of nicotine from 1 to 3 percent did not have a significant effect on the water activity of the liquid formulation.

Example 3: Aerosol Visibility

Various liquid formulations including nicotine, water, and/or propylene glycol are setforth below in Table 3. These examples were manufactured as set forth in Example 1.

Table 3

| Formulations | Composition w/w% | | | Test results | | |
|---|---|---|---|---|---|---|
|  | Nicotine | Water | Propylene glycol | Water Activity* | Boiling Point (°C)** | Viscosity mPa.s*** |
| Example 3A | 3.00 | 0.00 | 97.00 | 0.00 | 147.2 | 56,601 |
| Example 3B | 4.50 | 0.00 | 95.50 | 0.00 | 151.7 | 61,101 |
| Example 3C | 3.00 | 4.85 | 92.15 | 0.22 | 134.2 | 51,218 |
| Example 3D | 4.50 | 4.78 | 90.73 | 0.21 | 125.6 | 47,637 |
| Example 3E | 3.00 | 9.70 | 87.30 | 0.35 | 121.2 | 30,481 |
| Example 3F | 4.50 | 9.55 | 85.95 | 0.35 | 126.4 | 41,132 |
| Example 3G | 3.00 | 19.40 | 77.60 | 0.55 | 114.7 | 25,756 |
| Example 3H | 4.50 | 19.10 | 76.40 | 0.55 | 111.8 | 22,392 |
| Example 31 | 3.00 | 29.10 | 67.90 | 0.68 | 110.2 | 16,827 |
| Example 3J | 4.50 | 28.65 | 66.85 | 0.68 | 108.5 | 14,301 |
| Example 3K | 3.00 | 38.80 | 58.20 | 0.75 | 106.6 | 11,223 |
| Example 3L | 4.50 | 38.20 | 57.30 | 0.76 | 105.1 | 10,827 |

(continued)

| Formulations | Composition w/w% | | | Test results | | |
|---|---|---|---|---|---|---|
| | Nicotine | Water | Propylene glycol | Water Activity* | Boiling Point (°C)** | Viscosity mPa.s*** |
| Example 3M | 6 | 28.20 | 65.80 | 0.65 | 104.0 | 14,662 |
| Example 3N | 6 | 18.80 | 75.20 | 0.53 | 106.6 | 21,160 |
| Example 3O | 6 | 9.40 | 84.60 | 0.34 | 113.1 | 30,847 |
| * The water activity was measured by an AquaLab 4TEV apparatus. ** Boiling point was measured using the method described in the European Pharmacopoeia 8th Edition 2.2.8 *** Viscosity was measured using the method described in the European Pharmacopoeia 8th Edition 2.2.8 at 20°C | | | | | | |

These liquid formulations were tested with an aerosol-generating device composed of a cartomizer (Boge 510 Standard Resistance, Vaper Venue, Buena Park, CA) fitted to a battery (510 Titan Tank 340mAh Mega Automatic Battery, Totally Wicked Blackburn Lancashire, United Kingdom). About 0.8g of a formulation was filled into the liquid reservoir of the cartomizer, which was then fitted to the battery. The liquid formulations were tested for nicotine release using an *in vitro* puffing method. The aerosol generated by the device was captured and the nicotine level was determined. The result showed uniformity in nicotine release. For certain formulations in Table 3 more than 90% of the nicotine concentration of the formulation was measured in the captured aerosol.

All aerosols according to above Table 3 were less visible than the aerosols from a large number of commercial e-cigarettes. The aerosols according to Table 3 disappeared more rapidly upon dilution with the air. A slight additional decrease in aerosol visibility and duration was also noticed with increasing proportion of water in the formulations.

Example 4: Formulation Stability

The stability of Example 3N was followed during 12 weeks at three storage conditions: 25°C/60% RH (Table 4A), 40°C/75% RH (Table 4B), and 60°C ambient % RH (Table 4C). A gradient ultra-high performance liquid chromatography (UHPLC) method using a C18 column withstanding high pH and acetonitrile/ammonium acetate buffer as the eluent with UV detection at 230 to 260 nm. The results show an acceptable stability under these storage conditions.

Table 4A

| Attributes | Method | Specification Limits | Units | Storage time (weeks) | | | |
|---|---|---|---|---|---|---|---|
| | | | | 0 | 4 | 8 | 12 |
| Description[1] | Visual inspection | Informative | | Clear solution | Clear solution | Clear solution | Clear solution |
| Nicotine identity[2] | UHPLC | Rt matches standard | | Rt matches standard | Rt matches standard | Rt matches standard | Rt matches standard |
| Nicotine Assay[5] | UHPLC | 54.0-66.0 | mg/m1 | 61.7 | 61.4 | 62.7 | 62.5 |
| Impurities[3, 5] | | | | | | | |
| Cotinine | UHPLC | Informative | % l.c. | n.d | n.d | n.d | n.d |
| Nicotine-cis-N-oxide | UHPLC | Informative | % l.c. | n.d | n.d | n.d | n.d |
| Nicotine-trans-N-oxide | UHPLC | Informative | % l.c. | n.d | n.d | n.d | n.d |
| Norcotinine | UHPLC | Informative | % l.c. | n.d | n.d | n.d | n.d |
| Nornicotine | UHPLC | Informative | % l.c. | 0.11 | 0.13 | 0.11 | 0.10 |
| β-Nicotyrine | UHPLC | Informative | % l.c. | n.d | n.d | n.d | n.d |
| β-Nornicotyrine | UHPLC | Informative | % l.c. | n.d | n.d | n.d | n.d |

(continued)

| Attributes | Method | Specification Limits | Units | Storage time (weeks) | | | |
|---|---|---|---|---|---|---|---|
| | | | | 0 | 4 | 8 | 12 |
| Myosmine | UHPLC | Informative | % l.c. | n.d | 0,05 | 0,07 | 0,07 |
| MNP[4] | UHPLC | Informative | % l.c. | n.d | n.d | 0,03 | 0,04 |
| Any unspecified | UHPLC | Informative | % l.c. | n.d | n.d | n.d | n.d |
| Sum | UHPLC | Informative | % l.c. | 0.11 | 0.18 | 0.21 | 0.21 |

[1]Clear, colorless solution

[2] Conforms to test- retention time matches standard.

[3] No peaks smaller than the integration threshold (IT) are integrated, IT is defined as the area, which is 0.05 % of the nicotine peak area (the average peak area of the system suitability solution for nicotine). Peaks below IT are reported as n.d.

[4]1-methyl-3-nicotinoylpyrrolidine

[5] "% l.c." is the percent related substance of nicotine label claim, "n.a." and "n.d." stands for not analyzed and not detected, respectively

Table 4B

| Attributes | Method | Specification Limits | Units | Storage time (weeks) | | | |
|---|---|---|---|---|---|---|---|
| | | | | 0 | 4 | 8 | 12 |
| Description[1] | Visual inspection | Informative | | Clear solution | Clear solution | Clear solution | Clear solution |
| Nicotine identity[2] | UHPLC | Rt matches standard | | Rt matches standard | Rt matches standard | Rt matches standard | Rt matches standard |
| Nicotine Assay[5] | UHPLC | 54.0-66.0 | mg/ml | 61.7 | 62.0 | 62.5 | 61.8 |
| Impurities[3, 5] | | | | | | | |
| Cotinine | UHPLC | Informative | % l.c. | n.d | n.d | n.d | n.d |
| Nicotine-cis-N-oxide | UHPLC | Informative | % l.c. | n.d | n.d | 0.06 | 0.13 |
| Nicotine-trans-N-oxide | UHPLC | Informative | % l.c. | n.d | 0.09 | 0.14 | 0.25 |
| Norcotinine | UHPLC | Informative | % l.c. | n.d | n.d | n.d | n.d |
| Nornicotine | UHPLC | Informative | % l.c. | 0.11 | 0.13 | 0.10 | 0.12 |
| β-Nicotyrine | UHPLC | Informative | % l.c. | n.d | n.d | n.d | n.d |
| β-Nornicotyrine | UHPLC | Informative | % l.c. | n.d | n.d | n.d | n.d |
| Myosmine | UHPLC | Informative | % l.c. | n.d | 0.05 | 0.05 | 0.07 |
| MNP[4] | UHPLC | Informative | % l.c. | n.d | 0.04 | 0.04 | 0.04 |
| Any unspecified | UHPLC | Informative | % l.c. | n.d | n.d | n.d | n.d |

(continued)

| Attributes | Method | Specification Limits | Units | Storage time (weeks) | | | |
|---|---|---|---|---|---|---|---|
| | | | | 0 | 4 | 8 | 12 |
| Sum | UHPLC | Informative | % l.c. | 0.11 | 0.31 | 0.39 | 0.61 |

[1]Clear, colorless solution

[2] Conforms to test- retention time matches standard.

[3] No peaks smaller than the integration threshold (IT) are integrated, IT is defined as the area, which is 0.05 % of the nicotine peak area (the average peak area of the system suitability solution for nicotine). Peaks below IT are reported as n.d.

[4]1-methyl-3-nicotinoylpyrrolidine

[5] "% l.c." is the percent related substance of nicotine label claim, "n.a." and "n.d." stands for not analyzed and not detected, respectively

Table 4C

| Attributes | Method | Specification limits | Units | Storage time (weeks) | | | |
|---|---|---|---|---|---|---|---|
| | | | | 0 | 4 | 8 | 12 |
| Description[1] | Visual inspection | Informative | | Clear solution | Clear solution | Clear solution | Clear solution |
| Nicotine identity[2] | UHPLC | Rt matches standard | | Rt matches standard | Rt matches standard | Rt matches standard | Rt matches standard |
| Nicotine Assay[5] | UHPLC | 54.0-66.0 | mg/ml | 61.7 | 61.4 | 62.7 | 62.5 |
| Impurities[3, 5] | | | | | | | |
| Cotinine | UHPLC | Informative | % l.c. | n.d | n.d | 0.04 | 0.07 |
| Nicotine-cis-N-oxide | UHPLC | Informative | % l.c. | n.d | 0.21 | 0.46 | 0.74 |
| Nicotine-trans-N-oxide | UHPLC | Informative | % l.c. | n.d | 0.43 | 0.87 | 1.27 |
| Norcotinine | UHPLC | Informative | % l.c. | n.d | n.d | n.d | n.d |
| Nornicotine | UHPLC | Informative | % l.c. | 0.11 | 0.12 | 0.11 | 0.14 |
| β-Nicotyrine | UHPLC | Informative | % l.c. | n.d | n.d | n.d | n.d |
| β-Nomicotyrine | UHPLC | Informative | % l.c. | n.d | n.d | n.d | 0.02 |
| Myosmine | UHPLC | Informative | % l.c. | n.d | 0.07 | 0.08 | 0.09 |
| MNP[4] | UHPLC | Informative | % l.c. | n.d | 0.07 | 0.07 | 0.10 |
| Any unspecified | UHPLC | Informative | % l.c. | n.d | n.d | n.d | 0.11 |
| Sum | UHPLC | Informative | % l.c. | 0.11 | 0.90 | 1.63 | 2.54 |

[1]Clear, colorless solution

[2] Conforms to test- retention time matches standard.

[3] No peaks smaller than the integration threshold (IT) are integrated, IT is defined as the area, which is 0.05 % of the nicotine peak area (the average peak area of the system suitability solution for nicotine). Peaks below IT are reported as n.d.

[4]1-methyl-3-nicotinoylpyrrolidine

[5] "% l.c." is the percent related substance of nicotine label claim, "n.a." and "n.d." stands for not analyzed and not detected, respectively

Example 5: Optical Density of Aerosol

The optical densities of aerosols produced from two formulations were compared to aerosols produced by five commercially-available, nicotine aerosol-generating devices. The optical density of the aerosol produced by these seven formulations was tested in an *in vitro* model. For this model, a four liter cylindrical chamber was constructed having a radius of 12.5cm and the top and bottom constructed of clear polycarbonate to allow for the measurement of the optical density of the aerosol introduced into the chamber using a photometer (Hagner Photometer, Hagner Photometric Instruments Ltd., Havant, Sweden) positioned on one side of the chamber and a white light source positioned on the other side of the chamber. The chamber contains an aerosol introduction tube that forces air at a rate of 1 L/min for 3 seconds through the air inlet hole of the respective aerosol-generating devices and directs the resulting aerosol (50ml of aerosol) into the chamber. The chamber further contains both a fan (a 50mm fan running at approximately 2 CFM) to promote mixing and homogenization of introduced aerosol and a one way air check valve to both release any pressure increase within the chamber following introduction of the aerosol and allow flushing of the chamber in between each test.

For each device, the optical density was calculated using the following equation:

$$\text{Optical Density} = -\log_{10}(I/I_0) \quad \text{where } I = \text{light intensity and } I_0 = \text{initial light intensity}$$

Measurements were taken for 20 seconds following first instruction of the aerosol into the chamber (i.e., for the 3 seconds that the aerosol was introduced and for the seventeen seconds afterwards). The results of the studies are set forth below in Table 5.

Table 5

| Formulation | Optical Density | | | |
| --- | --- | --- | --- | --- |
| | 5 sec | 10 sec | 15 sec | 20 sec |
| Example 3F[1] | 0.002 | 0.000 | 0.002 | 0.002 |
| Example 3J[1] | 0.000 | 0.000 | 0.000 | 0.000 |
| Vype™ Classic Regular[2] | 0.087 | 0.087 | 0.092 | 0.092 |
| Vype™ Classic Bold[2] | 0.083 | 0.086 | 0.086 | 0.086 |
| Njoy™ King Gold[3] | 0.066 | 0.063 | 0.063 | 0.063 |
| Njoy™ King Bold[3] | 0.104 | 0.101 | 0.101 | 0.101 |
| Vuse™ Original[4] | 0.079 | 0.076 | 0.076 | 0.076 |
| 1 Aerosols of Examples 3F and 3J were created with a Joyetech 510-T body (Joyetech USA Inc, Irvine, CA) with Boge 510 cartomizer<br>2 Nicoventures Ltd., London, UK<br>3 Njoy, Scottsdale, AZ<br>4 RJ Reynolds Vapor, Salem, NC | | | | |

As is evident from the result in Table 5, Examples 3F and 3J produced unexpected, minimally persistent aerosols compared to tested commercial formulations. The Optical Density of Example 3F ranged between 0 and 0.002 while the Optical Density of Example 3J was 0 from 5-20 seconds. The five commercially available devices produced aerosols having much higher Optical Densities, ranging from 0.063 to 0.104 from 5-20 seconds.

It is understood that while the invention has been described in conjunction with the detailed description thereof, that the foregoing description is intended to illustrate and not limit the scope of the invention, which is defined by the scope of the appended claims.

Claims

1. A liquid formulation comprising:

    (i) 29.4 percent by weight of water;
    (ii) 68.6 percent by weight of propylene glycol; and

(iii) 2 percent by weight of nicotine.

2. A liquid formulation comprising:

(i) 39.2 percent by weight of water;
(ii) 58.8 percent by weight of propylene glycol; and
(iii) 2 percent by weight of nicotine.

3. A liquid formulation comprising:

(i) 28.8 percent by weight of water;
(ii) 67.2 percent by weight of propylene glycol; and
(iii) 4 percent by weight of nicotine.

4. A liquid formulation comprising:

(i) 38.4 percent by weight of water;
(ii) 57.6 percent by weight of propylene glycol; and
(iii) 4 percent by weight of nicotine.

5. A liquid formulation comprising:

(i) 29.10 percent by weight of water;
(ii) 67.90 percent by weight of propylene glycol; and
(iii) 3.00 percent by weight of nicotine.

6. A liquid formulation comprising:

(i) 28.65 percent by weight of water;
(ii) 66.85 percent by weight of propylene glycol; and
(iii) 4.50 percent by weight of nicotine.

7. A liquid formulation comprising:

(i) 38.80 percent by weight of water;
(ii) 58.20 percent by weight of propylene glycol; and
(iii) 3.00 percent by weight of nicotine.

8. A liquid formulation comprising:

(i) 38.20 percent by weight of water;
(ii) 57.30 percent by weight of propylene glycol; and
(iii) 4.50 percent by weight of nicotine.

9. A liquid formulation comprising:

(i) 28.20 percent by weight of water;
(ii) 65.80 percent by weight of propylene glycol; and
(iii) 6 percent by weight of nicotine.


**Patentansprüche**

1. Flüssige Formulierung, umfassend:

(i) 29,4 Gewichtsprozent Wasser;
(ii) 68,6 Gewichtsprozent Propylenglykol und
(iii) 2 Gewichtsprozent Nicotin.

**2.** Flüssige Formulierung, umfassend:

(i) 39,2 Gewichtsprozent Wasser;
(ii) 58,8 Gewichtsprozent Propylenglykol und
(iii) 2 Gewichtsprozent Nicotin.

**3.** Flüssige Formulierung, umfassend:

(i) 28,8 Gewichtsprozent Wasser;
(ii) 67,2 Gewichtsprozent Propylenglykol und
(iii) 4 Gewichtsprozent Nicotin.

**4.** Flüssige Formulierung, umfassend:

(i) 38,4 Gewichtsprozent Wasser;
(ii) 57,6 Gewichtsprozent Propylenglykol und
(iii) 4 Gewichtsprozent Nicotin.

**5.** Flüssige Formulierung, umfassend:

(i) 29,10 Gewichtsprozent Wasser;
(ii) 67,90 Gewichtsprozent Propylenglykol und
(iii) 3,00 Gewichtsprozent Nicotin.

**6.** Flüssige Formulierung, umfassend:

(i) 28,65 Gewichtsprozent Wasser;
(ii) 66,85 Gewichtsprozent Propylenglykol und
(iii) 4,50 Gewichtsprozent Nicotin.

**7.** Flüssige Formulierung, umfassend:

(i) 38,80 Gewichtsprozent Wasser;
(ii) 58,20 Gewichtsprozent Propylenglykol und
(iii) 3,00 Gewichtsprozent Nicotin.

**8.** Flüssige Formulierung, umfassend:

(i) 38,20 Gewichtsprozent Wasser;
(ii) 57,30 Gewichtsprozent Propylenglykol und
(iii) 4,50 Gewichtsprozent Nicotin.

**9.** Flüssige Formulierung, umfassend:

(i) 28,20 Gewichtsprozent Wasser;
(ii) 65,80 Gewichtsprozent Propylenglykol und
(iii) 6 Gewichtsprozent Nicotin.

**Revendications**

**1.** Formulation liquide comprenant :

(i) 29,4 pour cent en poids d'eau ;
(ii) 68,6 pour cent en poids de propylèneglycol ;
et
(iii) 2 pour cent en poids de nicotine.

**2.** Formulation liquide comprenant :

    (i) 39,2 pour cent en poids d'eau ;
    (ii) 58,8 pour cent en poids de propylèneglycol ;
    et
    (iii) 2 pour cent en poids de nicotine.

**3.** Formulation liquide comprenant :

    (i) 28,8 pour cent en poids d'eau ;
    (ii) 67,2 pour cent en poids de propylèneglycol ;
    et
    (iii) 4 pour cent en poids de nicotine.

**4.** Formulation liquide comprenant :

    (i) 38,4 pour cent en poids d'eau ;
    (ii) 57,6 pour cent en poids de propylèneglycol ;
    et
    (iii) 4 pour cent en poids de nicotine.

**5.** Formulation liquide comprenant :

    (i) 29,10 pour cent en poids d'eau ;
    (ii) 67,90 pour cent en poids de propylèneglycol ;
    et
    (iii) 3,00 pour cent en poids de nicotine.

**6.** Formulation liquide comprenant :

    (i) 28,65 pour cent en poids d'eau ;
    (ii) 66,85 pour cent en poids de propylèneglycol ;
    et
    (iii) 4,50 pour cent en poids de nicotine.

**7.** Formulation liquide comprenant :

    (i) 38,80 pour cent en poids d'eau ;
    (ii) 58,20 pour cent en poids de propylèneglycol ;
    et
    (iii) 3,00 pour cent en poids de nicotine.

**8.** Formulation liquide comprenant :

    (i) 38,20 pour cent en poids d'eau ;
    (ii) 57,30 pour cent en poids de propylèneglycol ;
    et
    (iii) 4,50 pour cent en poids de nicotine.

**9.** Formulation liquide comprenant :

    (i) 28,20 pour cent en poids d'eau ;
    (ii) 65,80 pour cent en poids de propylèneglycol ;
    et
    (iii) 6 pour cent en poids de nicotine.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- GB 2030862 A
- US 20130192617 A
- US 20140000638 A
- EP 1618803 A
- US 8127772 B

**Non-patent literature cited in the description**

- **ZHANG YAPING et al.** In vitro particle size distributions in electronic and conventional cigarette aerosols suggest comparable deposition patterns. *NICOTINE & TOBACCO RESEARCH: OFFICIAL JOURNAL OF THE SOCIETY FOR RESEARCH ON NICOTINE AND TOBACCO,* 01 February 2013, vol. 15 (2), 501-508